# NEW EUROPEAN PATENT SPECIFICATION

(11) **EP 0 553 244 B2**
(45) Date of publication and mention of the opposition decision: **06.04.2005**
(45) Mention of the grant of the patent: 30.12.1998
(21) Application number: 91919595.8
(22) Date of filing: 04.10.1991
(51) Int. Cl.: A61K 39/21, A61K 39/29, A61K 39/42, A61K 39/00, A61K 39/395, A61K 39/40

(54) **TARGETED IMMUNOSTIMULATION WITH BISPECIFIC REAGENTS**
GEZIELTE IMMUNOSTIMULIERUNG MIT BISPEZIFISCHEN STOFFEN
IMMUNOSTIMULATION CIBLEE INDUITE PAR DES REACTIFS BISPECIFIQUES

(30) Priority: 05.10.1990 US 593083
(43) Date of publication of application: 04.08.1993
(73) Proprietor: MEDAREX, INC., West Lebanon, NH 03784 (US); ROMET-LEMONNE, Jean Loup, F-91190 Gif-Yvette (FR)
(72) Inventor: ROMET-LEMONNE, Jean, Loup, F-91190 Gif-Yvette (FR); FANGER, Michael, W., Lebanon, NH 03766 (US)
(74) Representative: Kirkham, Nicholas Andrew
(86) International application number: PCT/US1991/007283
(87) International publication number: WO 1992/005793

(56) References cited:
- EP-A- 0 308 936
- WO-A-88/00052
- WO-A-91/00360
- US-A- 4 950 480
- SONGSIVILAI ET AL: 'A NOVEL STRATEGY FOR PRODUCING CHIMERIC BISPECIFIC ANTIBODIES BY GENE TRANSFECTION' BIOCHEMICAL AND BIOPHYSICAL RESEARCH COMMUNICATIONS vol. 164, 1989, NEW YORK,USA, pages 271 - 276, XP001194767
- CELIS ET AL: 'REGULATION OF T-CELL FUNCTION BY ANTIBODIES:ENHANCEMENT OF THE RESPONSE OF HUMAN T-CELL CLONES TO HEPATITIS B SURFACE ANTIGEN BY ANTIGEN-SPECIFIC MONOCLONAL ANTIBODIES' PROCEEDINGS OF THE NATIONAL ACADEMY OF SCIENCES,USA vol. 81, 1984, WASHINGTON D.C.,USA, pages 6846 - 6850, XP001194785
- Snider et al: 1990, Journal of Experimental Medicin, 171, 1957-1963
- Shen et al: 1986, Journal of Immunology, 137, 3378-3382
- Kawamura; Berzofsky: 1986, Journal of Immunology, 136, 58-65

## Description

### Background

Antigen molecules are recognized by the immune system after internal processing by antigen-presenting cells, generally mononuclear phagocytic cells such as macrophages. In order to present a proteinaceous antigen, the antigen-presenting cell first internalizes the antigen which is then broken down into small peptidic fragments by enzymes contained in vesicles in the cytoplasm of the antigen-presenting cells. After fragmentation, the peptides are linked to cellular major histocompatibility complex (MHC) molecules and presented on the presenting cell's surface to the immune system. Peptides presented in this way are recognized by the T-cell receptor which engages T-lymphocytes into the immune response against this antigen. This antigen presentation also stimulates the B lymphocytes for specific antibody production.

Complexes of antibody and antigen have been used to stimulate an immune response against the antigen. Antigen uptake through antigen-antibody conjugates bound to FcγR increases the efficiency of antigen presentation and thereby antigen-specific T-cell activation by human and mouse macrophages. Celis, E. and Chang, T.W. (1984) Science 224:297-299; Chang, T.W. (1985) Immunol. Today 6:245-259; Manca, R. et al. (1988) Immunol. 140:2893-2898; Schalke, B.C.G. et al. (1985) J. Immunol. 134:3643; and Snider, D.P. and Segal, D.M. (1987) J. Immunol. 139:1053-1059. The binding of these complexes to FcγR is mediated by the Fc region of the antibody. This binding is susceptible to inhibition by physiological levels of IgG.

WO 91/00360 is a document which is only available under Article 54(3) EPC. The document relates to:
bispecific molecules which react both with the high-affinity Fcγ receptor of human effector cells and with a virus or virus component. Binding of the molecules to the Fc receptors found on effector cells is not blocked by human immunoglobulin G. The molecules are useful for targeting human effector cells (e.g. macrophages) against a viral target (e.g. HIV or HIV-infected cell). For this purpose, bispecific molecules can be constructed containing the binding region derived from an anti-Fcγ receptor antibody and the CD4 molecule or CD4 binding domain of the envelope glycoprotein gp120 of HIV. Alternatively, bispecific antibodies or heteroantibodies can be constructed containing the binding region derived from an anti-Fc receptor antibody and the binding region of HIV-specific antibody such as anti-gp120 antibody. Targeted effector cells can be used to kill virus by cell mediated antibody dependent cytolysis.

US 4,950,480 discloses a method described for eliciting IgG antibody response to proteins or synthetic peptides without the requirement for the use of adjuvants, thereby making it easier and safer to confer protection against pathogenic organisms. The antigen is coupled to a monoclonal antibody, specific for membrane determinants expressed on certain types of mammalian recipient cells, called antigen presenting cells. The monoclonal antibody acts as a "vector" or "delivery vehicle" for targeting foreign antigens onto such recipient cells. This targeting facilitates subsequent antigen recognition by helper T-cells, which are pivotal in helping the induction of antigen-specific IgG responses.

J. Experimental Medicine, Vol. 171, June 1990, pages 1957-1963 (Snider et al) relates to enhanced antigen immunogenicity induced by bispecific antibodies. The cells recognise and respond to processed antigen bound to class II MHC molecules on the surfaces of APC (Chestnut et al., CRC Crit. Rev. Immunol. 5:236 (1985); Allen et al., Immunol. Rev. 98:171 (1987)). In vitro, the targeting of antigen to APC surfaces by heterocrosslinked bispecific antibodies (HBAs) greatly increases the efficiency with which APC endocytose, process, and present antigen to T cells (Snider et al., J Immunol. 139:1609 (1987); Snider et al., J. Immunol. 143:59 (1989), Ozaki et al., J. Immunol. 138:4133 (1987)). Since antibody responses against most protein antigens require T cell help in vivo, we have asked here if HBAs could also enhance the ability of an antigen, in this case hen egg lysozyme (HEL), to induce an antibody response in mice. HBAs were prepared by chemically crosslinking an antibody with specificity for HEL to various other antibodies, each specific for a particular APC cell surface component. Normally the generation of immune responses after immunisation with vaccines and other antigens requires relatively large amounts of antigen, multiple injections, and, in experimental animals, adjuvants (6,7). By contrast, we show that HBAs, when administered once with nanogram amounts of antigen, in the absence of adjuvant, induce high titers of antibody in mice, and prime mice for secondary IgG antibody response when rechallenged with soluble antigen.

### Summary of the Invention

The invention pertains to a molecular complex for stimulating an immune response to an antigen as claimed in claim 1. The present invention also pertains to a vaccine composition comprising the molecular complex in a pharmaceutically acceptable vehicle, and to the use of the molecular complex for the manufacture of a medicament for the treatment of a disease by stimulating the immune response against the antigen.

The bispecific binding agent specifically binds the Fc receptor of an antigen-presenting cell for immunoglobulin G (IgG) without being blocked by IgG. In a particularly preferred embodiment, the agent specifically binds the high affinity Fc receptor for immunoglobulin G (FcγRI).

The antigen to be targeted can be derived from a foreign pathogen or it can be derived from endogenous diseased host cells such as tumor cells. The antigen is administered as a preformed complex with the bispecific reagent.

The antigen is directly bound to a receptor-binding agent to create bispecific molecules. For example, the antigen can be covalently coupled to an antibody which binds the Fc receptor without being blocked by IgG.

The method and compositions of this invention can be used to treat or prevent infectious diseases, to neutralize the acute phase of an infection by a pathogenic organism, to stimulate the immune system in instances of chronic infection of such an organism and to treat tumors.

### Brief Description of the Figure

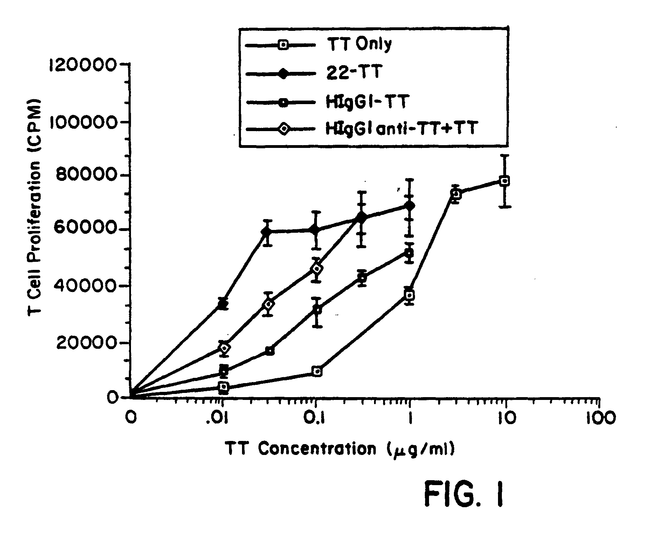

Figure 1 illustrates the enhanced antigen presentation by directing antigen to human FcγR.

### Detailed Description of the Invention

In the method of this invention, an antigen is targeted to an antigen-presenting cell to enhance the processes of internalization and presentation by these cells. The bispecific binding agent at the same time, binds a surface receptor of an antigen-presenting cell which can internalize antigen for processing and presentation. The receptor-binding component of the bispecific binding agent (and thus the bispecific binding agent itself) binds the receptor of the antigen-presenting cell without substantially being blocked by the natural ligand for the receptor. As a result, targeting of the antigen to the receptor will not be prevented by physiological levels of the ligand and the targeted receptor will remain capable of binding the ligand and functioning.

The preferred surface receptors of antigen-presenting cells for targeting are the receptors for the Fc region of IgG (FcγR). These receptors can mediate internalization of antibody-complexed antigens. The most preferred target is the high affinity Fc receptor (FcγRI). As described in more detail below, the bispecific binding agents are generally made of antibodies, antibody fragments or analogues of antibodies containing antibody-derived, antigen-binding (variable) regions. Antibodies that bind to Fc receptors on antigen-presenting cells, and whose binding to the receptor is not blocked by the natural ligand, can be produced by conventional monoclonal antibody methodology e.g., the standard somatic cell hybridization technique of Kohler and Milstein (1975) Nature 256:495. Although somatic cell hybridization procedures are preferred, in principle, other techniques for producing monoclonal antibodies can be employed e.g., viral or oncogenic transformation of B lymphocytes.

In general, an animal is immunized with an FcγR-bearing cell, a receptor-bearing portion thereof or the Fc receptor molecule in purified or partially purified form. Antibodies are selected which bind an epitope of the FcγR which is located outside of the ligand (i.e., Fc) binding domain of the receptor. This binding is not inhibited by IgG and, in turn, does not inhibit the binding of IgG and the function of the Fc receptor.

The production and characterization of monoclonal antibodies which bind FcγRI without being blocked by human IgG are described by Fanger et al. in PCT application WO 88/00052 and in U.S. Patent No. 4,954,617.

WO 88/00052 discloses the production of monoclonal antibodies as follows:

### Materials and Methods

### Chemicals and reagents

Cytochrome c Type VI, superoxide dismutase, pepstatin, chymostatin, leupeptin, antipain, rabbit muscle actin and phenylmethylsufonylfluoride (PMSF) were purchased from Sigma Chemical Co., St. Louis, MO; Dextran T500, Ficoll-Hypaque, Sepharose 4B, CNBr-activated Sepharose, Protein A-Sepharose CL-4B from Pharmacia Fine Chemicals, Piscataway, NJ; tetanus toxin, octyl-b-D-glucopyranoside (octyl-glucoside) and papain from Calbiochem, La Jolla, CA; human anti-tetanus toxin antibody (HyperTet™) from Cutter Laboratories, Berkeley, CA; chloroglycouril from Pierce Chemical Co., Rockford, IL; carrier-free I¹²⁵ (IMS.300) from Amersham, Arlington Heights, IL; cytochalasin B from Aldrich Chemical Co., Milwaukee, WI; goat F(ab')2 antimurine Ig (anti-mlg), both fluorescein isothiocyanate-conjugated (FITC) and unconjugated, from Cappel, West Chester, PA, unless indicated otherwise; RPMI 1640 from Gibco, Grand Island, NY, and from K C Biologicals, lenexa, KS; Fetal bovine serum (FBS) from Sterile Systems, Logan, UT; and a mixture of low molecular weight markers from Biorad, Richard, CA. Recombinant gamma interferon was kindly donated by Genentech, South San Francisco, CA. 1,25-dihydroxycholelcalciferol (1,25(OH)₂D₃) was a gift from Hoffran LaRoche, Nutley, NJ. Other chemicals were of analytical grade and were obtained commercially.

NP40 Lysis buffer contained 1% NP40, 110mM NaCl, 10mM EDTA, 2mM PMSF, 10ug/ml pepstatin, 10ug/ml chymostatin, 10ug/ml leupeptin and 10ug/ml antipain in 20 mM Tris buffer, pH 7.1. Krebs Ringer phosphate buffer with glucose (KRPglu) consisted of 135mM NaCl, 5mM KCl, 1.2mM MgS04, 1mM CaCl₂, 4.3mM glucose in 10mM sodium phosphate buffer, pH 7.4. Phosphate buffered saline (PBS) was 145mM NaCl in 20mM phosphate buffer, pH 7.0. PBS-K contained 130mM NaCl and 5mM KCI in 10mM Phosphate buffer, pH 7.4.

### Antibodies

The monoclonal antibody against the high affinity FcR (herein designated mab 32 and when subcloned, 32.2), was prepared as follows: A partially purified detergent lysate of the high affinity FcR from U937 cells was obtained in a manner similar to a published method (See Anderson, C.K., et al., (1984) J. Immunol. 134:465-470). U937 cells were lysed in 1% NP40 and the lysate was allowed to incubate with Sepharose hIgG for 8 hours. The adsorbent was washed thoroughly and was eluted with 0.5M acetic acid in 30mM octylglucoside. The eluate was promptly neutralised with 2M Tris and the amount of protein eluted was determined by Folin assay (Peterson, G.L. (1977) Anal. Biochem. 85:346-356). The tubes containing the bulk of the protein were pooled, concentrated by vacuum dialysis using an Amicon YM-10 filter and a Minicon apparatus to 0.5ml and emulsified with an equal volume of Freund's adjuvant, either complete for the first injection or incomplete for subsequent ones. A mouse was immunised intraperitoneally 4 times at roughly 4 week intervals, the last 2 immunisations using antigen derived from U937 cells cultured 72 hours in IFN-gamma, 100 IRU/ml, to increase the yield of FcR (Guyre, P.M., et al. (1983) J. Clin. Invest. 72:393-397). Five days following the last immunisation, the splenocytes were fused with cells of the NSI myeloma line by standard techniques (Kohler, G. and Milstein, C. (1975) Nature, 256:495; Ball, E.D., et al., (1982) PNAS 79:5374-5378). Supernatants of the hybrids were screened for their ability to bind to U937 cells by an indirect immuno-fluorescence assay using a flow cytometer. Chosen hybrids were cloned by limiting dilution, rescreened and expanded either in culture or in ascites fluid. The protein from clone mab 32 was found to be an IgGl antibody by an immunoblot assay using isotype-specific antisera. IgG of this clone was precipitated from ascites by making the solution 40% in ammonium sulfate. The precipitate was redissolved and dialysed against 20mM Tris buffer, pH 8.6. High performance ion exchange chromatography (HPLC) was carried out on a semi-preparative PROTEIN-PAK-5PW (Waters, Milford, MA) column. The initial eluting buffer was 20mM Tris, pH 8.6 delivered by pump A. 20mM Tris, 0.3M NaCl, pH 8.6 was delivered by pump B. A sixty minute linear gradient, 0-100% B, at a flow rate of 8 ml/min was used for elution. The main peak corresponding to IgG was pooled. For some experiments the purified IgG was passed over a SepharoseProtein A column to remove traces of IgG2a to less than 0.005%. Pepsin digestion of whole antibody was performed essentially as described by Parham (See Parham, P. (1983) J. Immunol. 131:2895-2902) except that the digestion time was 3 hrs and the pH 3.6. F(ab')2 was purified by high performance gel filtration chromatography using a TSK 250 column (Biorad). Fab' was made from F(ab')2 by reducing with 1 mM dithiothreitol for 1 hr at room temperature and alkylating with an excess of iodoacetamide. The Fab' was purified by HPLC using the TSK 250 column.

The preparation of mab 22, mab 44, mab 62 and mab 197 were as above, except that for mabs 22, 44 and 197 the immuogen was IFN-gamma- and dexamethasone activated U937 cells. All procedures and preparations were the same as for mab 32.

These antibodies bind to an epitope of FcγRI which is distinct from the Fc binding site of the receptor and, thus, their binding is not blocked substantially by physiological levels of IgG. Specific anti-FcγRI antibodies useful in this invention are mab 22, mab 32, mab 44, mab 62 and mab 197. The hybridoma producing mab 32 is available from the American Type Culture Collection, Rockville, MD, ATCC No. HB9469.

Bispecific antibodies are single, divalent antibodies which have two different antigen binding sites (variable regions). These antibodies can be produced by chemical techniques (see e.g., Kranz, D.M. et al. (1981) Proc. Natl. Acad. Sci. USA 78:5807), by "polydoma" techniques (See U.S. Patent 4,474,893, to Reading) or by recombinant DNA techniques.

Heteroantibodies are two or more antibodies or antibody-binding fragments (Fv, Fab, Fab' or F(ab')₂) of different binding specificity linked together. Heteroantibodies can be prepared by conjugating together two or more antibodies or antibody fragments. Preferred heteroantibodies are comprised of crosslinked Fab fragments. A variety of coupling or cross linking agents can be used to conjugate the antibodies. Examples are protein A, carboiimide, N-Succinimidyl-S-acetyl-thioacetate (SATA) and N-succinimidyl-3-(2-pyridyldithio) propionate (SPDP). See e.g., Karpovsky et al. (1984) J. Exp. Med. 160:1686; Liu, M.A. et al. (1985) Proc. Natl. Acad. Sci. USA 82:8648. Other methods include those described by Paulus, H. Behring Inst. Mitt., No. 78, 118-132 (1985); Brennan et al. (1985) Science 229:81-83 or Glennie et al. (1987), J. Immunol. 139:2367-2375.

Bispecific binding agents can also be prepared from single chain antibodies. See e.g., Huston, J.S. et al. (1988) Proc. Natl. Acad. Sci. 85:5879; Skerra, A. and Plucthun, A. (1988) Science 240:1038. These are analogues of antibody variable regions produced as a single polypeptide chain. To form the bispecific binding agent, the single chain antibodies may be coupled together chemically or by genetic engineering methods.

As used herein, the term antigen means any natural or synthetic antigenic substance, a fragment or portion of an antigenic substance, a peptidic epitope, or a hapten. Suitable antibodies against wide variety of antigens for construction of the bispecific binding agents are available or can be readily made by standard techniques.

In some cases, it may be desirable to couple a substance which is weakly antigenic or nonantigenic in its own right (such as a hapten) to a carrier molecule, such as a large immunogenic protein (e.g., a bacterial toxin) for administration. In these instances, the bispecific binding reagent can be made to bind an epitope of the carrier to which the substance is coupled, rather than an epitope of the substance itself.

In another embodiment of the invention, the antigen can be coupled directly to the binding agent for the receptor. For example, an antigen can be coupled to an antibody, or fragment thereof, specific for an Fc receptor of an antigen-presenting cell. Proteinaceous antigens can be coupled by the methods described above or by other methods.

The antigen targeted by the method of this invention can be soluble or particulate; it may carry B cell epitopes, T cell epitopes or both. The antigen can be bacterial, viral or parasitic in origin. Often, the antigen will comprise a component of the surface structure of a pathogenic organism. For example, the antigen can comprise a viral surface structure such as an envelope glycoprotein of human immunodeficiency virus (HIV) or the surface antigen of hepatitis virus. In addition, the antigen can be associated with a diseased cell, such as a tumor cell, against which an immune response may be raised for treatment of the disease. The antigen can comprise a tumor-specific or tumor-associated antigen, such as the Her-2/neu proto-oncogene product which is expressd on human breast and ovarian cancer cells (Slamon, D.J. et al. (1989) Science 244:707).

Targeted immunostimulation can be performed in vitro or in vivo. The bispecific binding agent can be used to target an antigen to antigen-presenting cells in culture. Immunocompetent cells are separated and purified from patient blood. The cells are exposed to the antigen and the binding agent. Targeted antigen-presenting cells will process the antigen and present fragments on their surface. After stimulation, the cells can be returned to the patient.

To elicit an immune response in vivo, the antigen is administered to a host in conjunction with the binding agent. The complex is formed by incubating the antigen and the bispecific binding agent at a desired molar ratio under conditions which permit binding of the two species.

The complex is administered in a physiologically acceptable solution at a dosage which will evoke an immune response against the antigen. The optimum dose of antigen, as well as the molar ratio of antigen and binding agent, may vary dependent upon factors such as the type of antigen, the immune status of the host, the type of infection or other disease being treated, etc. In most cases, the dose of antigen required to elicit an immune response (as determined by any standard method for assessment of immune response) should be lower than that which would be required if the antigen were given alone or as a complex with a monospecific antibody for the antigen.

The method of this invention can be used to enhance or reinforce the immune response to an antigen. For example, the method is valuable for the treatment of chronic infections, such as hepatitis and AIDS, where the unaided immune system is unable to overcome the infection. It can also be used in the treatment of the acute stages of infection when reinforcement of immune response against the invading organism may be necessary.

The method can be used to reduce the dose of antigen required to obtain a protective or therapeutic immune response or in instances when the host does not respond or responds minimally to the antigen. Although generally desirable, the lowering of effective dose can be especially desirable when the antigen is toxic to the host.

The method of targeted immunostimulation can also be used in disease therapy. For example, the bispecific binding agent can be used to target a tumor-associated (or tumor-specific) antigen to an antigen-presenting cell in order to cause or to enhance an immune response against the tumor.

The invention is illustrated further by the following exemplification:

### Exemplification

### Example 1 (Referential)

A bispecific heteroantibody was prepared from a monoclonal antibody against human erythrocytes (mono-D, a human anti-RhD antibody) and anti-FcγRI antibody 32, by a protocol previously described. Shen, C. et al. (1986) J. Immunol. 137:3378. Human erythrocytes were washed three times in buffer solution and then incubated for 60 minutes at 37°C in solution of the heteroantibody. After the incubation and three washings, erythrocytes coated with heteroantibody were diluted at 5x10⁷ cells per millimeter in Hank's buffer and then incubated with adherent monocytes (macrophages) at the ratio of 100:1 for one hour at 37°C. Cells were then washed in phosphate buffered saline (PBS), fixed for one minute in ethanol and stained with Giemsa for observation through a light microscope.

Internalization of erythrocytes was easily observed as unstained spheres in the macrophage cytoplasm. The number of macrophages that internalized at least one erythrocyte were counted. This experiment was repeated numerous times with and without the heteroantibody present. In each experiment, no erythrocyte internalization was observed in macrophages which were incubated with erythrocytes in the absence of the heteroantibody.

In addition, experiments were performed after treatment of adherent monocytes (macrophages) with various concentrations of interferon-gamma which is known to increase the number of FcγRI receptors on the macrophage surface. Petroni, K.C, et al. (1988) J. Immunol. 140:3467. As shown in the table below, the number of macrophages that internalized erythrocytes increased in a direct relation to the concentration of interferon-gamma.

**Table**

| Gamma Interferon Concentration (µg/ml) | Percentage of Macrophages Having Internalized at Least One Erythrocyte (%) |
|---|---|
| 1000 | 40 |
| 100 | 25 |
| 10 | 6 |

These data show that the heteroantibody can trigger internalization of antigen by macrophages.

### Example 2 Enhanced Tetanus Toxoid (TT) Presentation by directing TT to human FcγR.

Monoclonal antibody 22 (mAb 22) is specific for the high affinity Fcγ receptor and its binding to the receptor is not blocked by IgG Fc. See U.S. Patent No. 4,954,617. TT was conjugated to F(ab')₂ of mAb 22. To test the potential role of human antibody (Ab) isotype, TT was conjugated to non-specific HlgG'. TT (obtained from Accurate Chemical Co., Westburg, NY) was linked to antibody or antibody fragments by the SATA-malemide procedure.

The experiments were done in serum free AIM V medium (Gibco, Grand Island, NY) to minimize the contribution of undefined components such as hormones, lymphokines or monomeric and polymeric immunoglobulins. The use of AIM V reduces non-specific T cell responses while maintaining Ag-specific responses equal to those observed with other media tested. This medium allows more definitive studies of Fc receptor-enhanced antigen presentation in vitro. If antigen is directed to Fc receptors using mAb that bind to Fc receptors regardless of the presence of human IgG, this medium is not a requirement to see enhanced Ag presentation.

T cells used in the assay were primed with TT. When T cells are taken fresh from an individual there are T cells present which can potentially respond to many things (serum components, mouse Ig, etc.). By priming the cells in vitro (i.e., adding TT to fresh monocytes and T cells), only the T cells which recognize TT grow out. Thus, the cells are specific for TT.

The T cells were taken from the same donor as the monocytes. The vast majority (>85%) are CD4+, helper T cells specific for TT. They are polyclonal which means they likely recognize many parts of TT (i.e., many different 10-20 amino acid segments of TT as foreign). This is the type of response (polyclonal) which one might expect in vivo.

5 x 10⁴ monocytes purified by cold aggregation and 5 x 10⁴ T cells (primed once with TT, as described) were added in AIM V medium to wells of a 96 well plate. Subsequently, A, TT, TT-Ab, or anti-TT A + TT was added. Plates were incubated 72 hrs at 37°C at which time [³H]thymidine was added overnight. Cells were then harvested and counted.

Figure 1 shows the results of these experiments. Data is expressed as counts/minute (CPM) ± SD. As can be seen, TT conjugated to mAb 22 resulted in enhanced T cell proliferation over that obtained with TT alone, HlgGl-TT or anti-TT:TT complex. Ab alone did not induce T cell proliferation.

### Equivalents

Those skilled in the art will recognize, or be able to ascertain using no more than routine experimentation, many equivalents to the specific embodiments of the invention described herein. Such equivalents are intended to be encompassed by the following claims.

## Claims (Claims for the following Contracting State(s): AT, BE, CH/LI, DE, DK, FR, GB, IT, LU, NL, SE)

1. A molecular complex for stimulating an immune response to an antigen, comprising the antigen covalently linked to a binding agent which binds to a surface receptor of an antigen-presenting cell without being blocked substantially by the natural ligand for the receptor, wherein the surface receptor of the antigen presenting cell is an Fc receptor.

2. The molecular complex of claim 1, wherein the antigen is selected from a viral, a bacterial, a parasitic and a tumour-associated antigen.

3. The molecular complex of claim 1 or 2, wherein the antigen is selected from the group comprising: a hepatitis antigen, optionally a hepatitis B antigen; a hepatitis surface antigen; an HIV antigen; a tetanus toxin; a breast cancer-associated antigen; an ovarian cancer-associated antigen optionally a Her-2/neu.

4. The molecular complex of claim 1 wherein the Fc receptor is an Fcγ receptor.

5. The molecular complex of any one of the preceding claims, wherein the antigen presenting cell is a mononuclear phagocyte derived antigen presenting cell.

6. The molecular complex of claim 1, wherein the antigen binding site specific for an Fc receptor for immunoglobulin G is from monoclonal antibody 32 produced by the hybridoma having the ATCC Deposit Number HB9469.

7. The molecular complex according to any one of claims 1 to 6, wherein one antigen binding site is produced recombinantly.

8. The molecular complex according to any one of the claims 1 to 7, wherein the antigen binding sites are in antibodies or fragments thereof which are chemically coupled.

9. The molecular complex according to any one of the preceding claims wherein the binding agent is produced recombinantly.

10. The molecular complex according to any one of the preceding claims which is produced recombinantly.

11. The molecular complex according to any one of the claims for use in therapy.

12. A vaccine composition, comprising the molecular complex according to any one of the preceding claims in a pharmaceutically acceptable vehicle.

13. Use of the molecular complex according to any one of claims 1 to 11 for the manufacture of a medicament for the treatment of a disease by stimulating the immune response against the antigen.

## Claims (Claims for the following Contracting State(s): ES, GR)

1. A method of preparing a molecular complex for stimulating an immune response to an antigen, comprising the antigen covalently linked to a binding agent which binds to a surface receptor of an antigen-presenting cell without being blocked substantially by the natural ligand for the receptor, wherein the surface receptor of the antigen presenting cell is an Fc receptor.

2. The method of claim 1, wherein the antigen is selected from a viral, a bacterial, a parasitic and a tumour-associated antigen.

3. The method of claim 1 or 2, wherein the antigen is selected from the group comprising: a hepatitis antigen, optionally a hepatitis B antigen; a hepatitis surface antigen; an HIV antigen; a tetanus toxin; a breast cancer-associated antigen; an ovarian cancer-associated antigen optionally a Her-2/neu.

4. The method of claim 1, wherein the Fc receptor is an Fcγ receptor.

5. The method of any one of the preceding claims, wherein the antigen presenting cell is a mononuclear phagocyte derived antigen presenting cell.

6. The method of claim 1, wherein the antigen binding site specific for an Fc receptor for immunoglobulin G is from monoclonal antibody 32 produced by the hybridoma having the ATCC Deposit Number HB9469.

7. The method according to any one of claims 1 to 6, wherein one antigen binding site is produced recombinantly.

8. The method according to any one of the claims 1 to 7, wherein the antigen binding sites are in antibodies or fragments thereof which are chemically coupled.

9. The method according to any one of the preceding claims wherein the binding agent is produced recombinantly.

10. The method according to any one of the preceding claims which is produced recombinantly.

11. The method according to any one of the claims for use in therapy.

12. A vaccine composition, comprising the molecular complex producible by the method any one of the preceding claims in a pharmaceutically acceptable vehicle.

13. Use of a molecular complex producible by the method according to any one of claims 1 to 11 for the manufacture of a medicament for the treatment of a disease by stimulating the immune response against the antigen.

## Patentansprüche (Patentansprüche für folgende(n) Vertragsstaat(en): AT, BE, CH/LI, DE, DK, FR, GB, IT, LU, NL, SE)

1. Ein Molekülkomplex zur Stimulierung einer Immunantwort auf ein Antigen, der umfaßt: das Antigen, das an ein bindendes Agens gekoppelt ist, das an einen oberflächenrezeptor einer Antigen-prasentierenden Zelle bindet, ohne wesentlich durch den natürlichen Liganden für den Rezeptor blockiert zu sein, worin der Oberflächenrezeptor der Antigen-präsentierenden Zelle ein Fc-Rezeptor ist.

2. Der Molekülkomplex gemäß Anspruch 1, worin das Antigen aus einem viralen, einem bakteriellen, einem parasitischen und einem Tumor-assoziierten Antigen gewählt ist.

3. Der Molekülkomplex gemäß Anspruch 1 oder 2, worin das Antigen aus der Gruppe gewählt ist umfassend: ein Hepatitis-Antigen, wahlweise ein Hepatitis-B-Antigen; ein Hepatitis-Oberflächenancigen; ein HIV-Antigen; ein Tetanustoxin; ein Brustkrebskarzinom-assoziiertes Antigen; ein Ovarialkarzinom-assoziiertes Antigen, wahlweise ein Her-2/neu.

4. Der Molekülkomplex nach Anspruch 1, worin der Fc-Rezeptor ein Fcγ-Rezeptor ist.

5. Ein Molekülkomplex nach einem der vorausgehenden Ansprüche, worin die Antigen-präsentierende Zelle eine Antigen-präsentierenden Zelle ist, die von einem mononuklearen Phagocyt abstammt.

6. Der Molekülkomplex nach Anspruch 1, worin die für einen Fc-Rezeptor für Immunglobulin G spezifische Antigenbindungsstelle vom monoklonalen Antikörper 32 ist, der von dem Hybridom mit der ATCC Hinterlegungsnummer HB9469 produziert wird.

7. Der Molekülkomplex gemäß einem der Ansprüche 1 bis 6, worin eine Antigenbindungsstelle rekombinant produziert ist.

8. Der Molekülkomplex gemäß einem der Ansprüche 1 bis 7, worin die Antigenbindungsstellen in Antikörpern oder Fragmenten davon sind, die chemisch gekoppelt sind.

9. Der Molekülkomplex gemäß einem der vorausgehenden Ansprüche, worin das bindende Agens rekombinant hergestellt ist.

10. Der Molekülkomplex gemäß einem der vorausgehenden Ansprüche, der rekombinant hergestellt ist.

11. Ein Molekülkomplex gemäß einem der vorausgehenden Ansprüche zur therapeutischen verwendung.

12. Eine Impfstoff-Zusammensetzung, die einen Molekülkomplex nach einem der vorausgehenden Ansprüche in einem pharmazeutisch geeigneten Träger umfaßt.

13. Verwendung eines Molekülkomplexes gemäß einem der Ansprüche 1 bis 11 zur Herstellung eines Medikaments zur Behandlung einer Krankheit durch Stimulation der Immunantwort gegen das Antigen.

## Patentansprüche (Patentansprüche für folgende(n) Vertragsstaat(en): ES, GR)

1. Ein Verfahren zur Herstellung eines Molekülkomplexes zur Stimulierung einer Immunantwort auf ein Antigen umfassend das Antigen kovalent gebunden an ein bindendes Agens, das an einen Oberflächenrezeptor einer Antigen-präsentierenden Zelle bindet, ohne wesentlich durch den natürlichen Liganden für den Rezeptor blockiert zu sein, worin der Oberflächenrezeptor der Antigen-präsentierenden Zelle ein Fc-Rezeptor ist.

2. Das Verfahren gemäß Anspruch 1, worin das Antigen aus einem viralen, einem bakteriellen, einem parasitischen und einem Tumor-assoziierten Antigen gewählt ist.

3. Das Verfahren gemäß Anspruch 1 oder 2, worin das Antigen aus der Gruppe gewählt ist umfassend: ein Hepatitis-Antigen, wahlweise ein Hepatitis-B-Antigen; ein Heptatitis-Oberflächenantigen; ein HIV-Antigen; ein Tetanustoxin; ein Brustkrebskarzinom-assoziiertes Antigen; ein Ovarialkarzinom-assoziiertes Antigen, wahlweise ein Her-2/neu.

4. Das Verfahren nach Anspruch 1, worin der Fc-Rezeptor ein Fcγ-Rezeptor ist.

5. Das Verfahren nach einem der vorausgehenden Ansprüche, worin die Antigen-präsentierende Zelle eine Antigen-präsentierende Zelle ist, die von einem mononuklearen Phagocyt abstammt.

6. Das Verfahren nach Anspruch 1, worin die für einen Fc-Rezeptor für Immunglobulin G spezifische Antigenbindungsstelle von einem monoklonalen Antikörper 32 ist, der vom Hybridom mit der ATCC Hinterlegungsnummer HB9469 produziert wird.

7. Das verfahren gemäß einem der Ansprüche 1 bis 6, worin eine Antigenbindungsstelle rekombinant produziert ist.

8. Das Verfahren gemäß einem der Ansprüche 1 bis 7, worin die Antigenbindungsstellen in Antikörpern oder Fragmenten davon sind, die chemisch gekoppelt sind.

9. Das Verfahren gemäß einem der vorausgehenden Ansprüche, worin das bindende Agens rekombinant hergestellt ist.

10. Das Verfahren gemäß einem der vorausgehenden Ansprüche, das rekombinant hergestellt ist.

11. Das Verfahren gemäß einem der vorausgehenden Ansprüche zur therapeutischen Verwendung.

12. Eine Impfstoff-Zusammensetzung, die einen mit dem Verfahren nach einem der vorausgehenden Ansprüche herstellbaren Molekülkomplex in einem pharmazeutisch geeigneten Träger umfaßt.

13. Verwendung eines Molekülkomplexes, der mit dem Verfahren nach einem der Ansprüche 1 bis 11 herstellbar ist, zur Herstellung eines Medikaments zur Behandlung einer Krankheit durch Stimulation der Immunantwort gegen das Antigen.

## Revendications (Revendications pour l'(les) Etat(s) contractant(s) suivant(s): AT, BE, CH/LI, DE, DK, FR, GB, IT, LU, NL, SE)

1. Complexe moléculaire destiné à stimuler une réponse immunitaire à un antigène comprenant : l'antigène couplé à un agent de liaison, qui se lie à un récepteur de surface d'une cellule de présentation antigénique sans être notablement inhibé par le ligand naturel pour le récepteur, le récepteur de surface de la cellule de présentation antigénique étant un récepteur pour Fc.

2. Complexe moléculaire selon la revendication 1, dans lequel l'antigène est choisi parmi un antigène viral, un antigène bactérien, un antigène parasitaire et un antigène associé à une tumeur.

3. Complexe moléculaire selon la revendication 1 ou 2, dans lequel l'antigène est choisi dans le groupe formé par : un antigène associé à l'hépatite, facultativement un antigène associé à l'hépatite B ; un antigène de surface associé à l'hépatite ; un antigène de VIH ; une toxine tétanique ; un antigène associé au cancer du sein ; un antigène associé au cancer ovarien, facultativement Her-2/neu.

4. Complexe moléculaire selon la revendication 1, dans lequel le récepteur pour Fc est un récepteur pour Fcγ.

5. Complexe moléculaire de l'une quelconque des revendications précédentes, dans lequel la cellule de présentation antigénique est une cellule de présentation antigénique dérivée de phagocyte mononucléaire.

6. Complexe moléculaire selon la revendication 1, dans lequel le site de liaison d'antigène spécifique envers un récepteur pour le Fc d'immunoglobuline G provient d'un anticorps monoclonal 32 produit par l'hybridome ayant le numéro de dépôt ATCC HB9468.

7. Complexe moléculaire selon l'une quelconque des revendications 1 à 6, dans lequel un seul site de liaison d'antigène est produit par voie de recombinaison.

8. Complexe moléculaire selon l'une quelconque des revendications 1 à 7, dans lequel les sites de liaison d'antigène se trouvent dans des anticorps ou des fragments de ceux-ci qui sont couplés chimiquement.

9. Complexe moléculaire selon l'une quelconque des revendications précédentes, dans lequel l'agent de liaison est produit par voie de recombinaison.

10. Complexe moléculaire selon l'une quelconque des revendications précédentes, qui est produit par voie de recombinaison.

11. Complexe moléculaire selon l'une quelconque des revendications précédentes, pour son utilisation en thérapie.

12. Composition de vaccin, comprenant un complexe moléculaire selon l'une quelconque des revendications précédentes dans un véhicule pharmaceutiquement acceptable.

13. Utilisation d'un complexe moléculaire selon l'une quelconque des revendications 1 à 11, pour la fabrication d'un médicament destiné au traitement d'une maladie par stimulation de la réponse immunitaire contre l'antigène.

## Revendications (Revendications pour l'(les) Etat(s) contractant(s) suivant(s): ES, GR)

1. Procédé de préparation d'un complexe moléculaire destiné à stimuler une réponse immunitaire à un antigène comprenant : l'antigène couplé à un agent de liaison, qui se lie à un récepteur de surface d'une cellule de présentation antigénique sans être notablement inhibé par le ligand naturel pour le récepteur, le récepteur de surface de la cellule de présentation antigénique étant un récepteur pour Fc.

2. Procédé selon la revendication 1, dans lequel l'antigène est choisi parmi un antigène viral, un antigène bactérien, un antigène parasitaire et un antigène associé à une tumeur.

3. Procédé selon la revendication 1 ou 2, dans lequel l'antigène est choisi dans le groupe formé par : un antigène associé à l'hépatite, facultativement un antigène associé à l'hépatite B ; un antigène de surface associé à l'hépatite ; un antigène de VIH ; une toxine tétanique ; un antigène associé au cancer du sein : un antigène associé au cancer ovarien, facultativement Her-2/neu.

4. Procédé selon la revendication 1, dans lequel le récepteur pour Fc est un récepteur pour Fcγ.

5. Procédé selon l'une quelconque des revendications précédentes, dans lequel la cellule de presentation antigénique est une cellule de présentation antigénique dérivée de phagocyte mononucléaire.

6. Procédé selon la revendication 1, dans lequel le site de liaison d'antigène spécifique envers un récepteur pour le Fc d'immunoglobuline G provient d'un anticorps monoclonal 32 produit par l'hybridome ayant le numéro de dépôt ATCC HB9469.

7. Procédé selon l'une quelconque des revendications 1 à 6, dans lequel un seul site de liaison d'antigène est produit par voie de recombinaison.

8. Procécié selon l'une quelconque des revendications 1 à 7, dans lequel les sites de liaison d'antigène se trouvent dans des anticorps ou des fragments de ceux-ci qui sont couplés chimiquement.

9. Procédé selon l'une quelconque des revendications précédentes, dans lequel l'agent de liaison est produit par voie de recombinaison.

10. Procédé selon l'une quelconque des revendications précédentes, qui est produit par voie de recombinaison.

11. Procédé selon l'une quelconque des revendications précédentes, pour son utilisation en thérapie.

12. Composition de vaccin, comprenant un complexe moléculaire pouvant être produit par le procédé selon l'une quelconque des revendications précédentes, dans un véhicule pharmaceutiquement acceptable.

13. Utilisation d'un complexe moléculaire pouvant être produit par le procédé selon l'une quelconque des revendications 1 à 11, pour la fabrication d'un médicament destiné au traitement d'une maladie par stimulation de la réponse immunitaire contre l'antigène.
